(19) Europäisches Patentamt
European Patent Office
Office européen des brevets

(11) Publication number: **0 306 826**
**A1**

(12) **EUROPEAN PATENT APPLICATION**

(21) Application number: **88114249.1**

(22) Date of filing: **01.09.88**

(51) Int. Cl.[4]: **C07D 219/10 , C07D 221/16 , C07D 221/22 , C07D 495/04 , A61K 31/47 , //(C07D495/04, 219:00,333:00),(C07D495/04, 219:00,335:00)**

Claims for the following Contracting States: ES + GR.

(30) Priority: **08.09.87 US 94181**
**25.07.88 US 223848**

(43) Date of publication of application:
**15.03.89 Bulletin 89/11**

(84) Designated Contracting States:
**AT BE CH DE ES FR GB GR IT LI LU NL SE**

(71) Applicant: **HOECHST-ROUSSEL PHARMACEUTICALS INCORPORATED**
**Route 202-206 North**
**Somerville New Jersey 08876(US)**

(72) Inventor: **Shutske, Gregory Michael**
**47 Cedarbrook Drive**
**Somerset, N.J. 08873(US)**
Inventor: **Kapples, Kevin James**
**P.O. Box 202**
**Little York, N.J. 08834(US)**

(74) Representative: **Becker, Heinrich Karl Engelbert, Dr. et al**
**HOECHST AKTIENGESELLSCHAFT Central Patent Department P.O. Box 80 03 20**
**D-6230 Frankfurt am Main 80(DE)**

(54) **N-(substituted alkylidene)fused bicycloalkylidene and hetero-alkylidene quinolinamines, a process for their preparation and their use as medicaments.**

(57) There are disclosed compounds having the formula

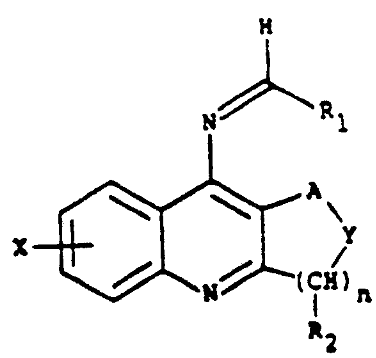

wherein n is 1-4; $R_1$ is hydrogen, alkyl, aryl, arylloweralkyl, naphthyl, furyl, thienyl, pyridinyl or pyrrolyl; A is a direct bond or $(CHR_3)_m$, m being 1-3; X is hydrogen, loweralkyl, cycloalkyl, loweralkoxy, halogen, hydroxy, nitro, trifluoromethyl, formyl, loweralkylcarbonyl, arylcarbonyl, -SH, loweralkylthio, $-NHCOR_4$ or $-NR_5R_6$, $R_4$ being hydrogen or loweralkyl, and $R_5$ and $R_6$ being independently hydrogen, loweralkyl or cycloalkyl; Y is $CH_2$, O, S or $NR_7$; and each $R_2$, each $R_3$ and $R_7$ are independently hydrogen, loweralkyl or arylloweralkyl

EP 0 306 826 A1

or taken two at a time form a methylene or ethylene bridge constituting a part of a five-membered or larger ring, with the proviso that when Y is $CH_2$, $R_2$ and $R_3$ must together constitute a methylene or ethylene bridge; stereo, optical and geometrical isomers thereof, and pharmaceutically acceptable acid addition salts thereof, which are useful for enhancing memory.

# N-[Substituted alkylidene]fused-bicycloalkylidene and heteroalkylidene quinolinamines, a process for their preparation and their use as medicaments

This invention relates to compounds having the formula

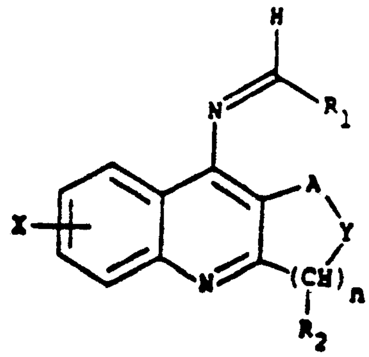

wherein n is 1-4; $R_1$ is hydrogen, alkyl, aryl, arylloweralkyl, naphthyl, furyl, thienyl, pyridinyl or pyrrolyl; A is a direct bond or $(CHR_3)_m$, m being 1-3; X is hydrogen, loweralkyl, cycloalkyl, loweralkoxy, halogen, hydroxy, nitro, trifluoromethyl, formyl, loweralkylcarbonyl, arylcarbonyl, -SH, loweralkylthio, $-NHCOR_4$ or $-NR_5R_6$, $R_4$ being hydrogen or loweralkyl, and $R_5$ and $R_6$ being independently hydrogen, loweralkyl or cycloalkyl; Y is $CH_2$, O, S or $NR_7$; and each $R_2$, each $R_3$ and $R_7$ are independently hydrogen, loweralkyl or arylloweralkyl or taken two at a time form a methylene or ethylene bridge constituting a part of a five-membered or larger ring, with the proviso that when Y is $CH_2$, $R_2$ and $R_3$ must together constitute a methylene or ethylene bridge; stereo, optical and geometrical isomers thereof, and pharmaceutically acceptable acid addition salts thereof, which are useful for enhancing memory.

Throughout the specification and the appended claims, a given chemical formula or name shall encompass all stereo and optical isomers thereof where such isomers exist, as well as pharmaceutically acceptable acid addition salts thereof and solvates thereof such as for instance hydrates.

The following definitions shall apply throughout the specification and the appended claims.

Unless otherwise stated or indicated, the term alkyl denotes a straight or branched alkyl group having from 1 to 18 carbon atoms. Examples of said alkyl include methyl, n-propyl, iso-butyl, heptyl, decyl, dodecyl, hexadecyl and octadecyl.

Unless otherwise stated or indicated, the term loweralkyl denotes a straight or branched alkyl group having from 1 to 6 carbon atoms. Examples of said loweralkyl include methyl, ethyl, n-propyl, iso-butyl, pentyl and hexyl.

Unless otherwise stated or indicated, the term cycloalkyl denotes a saturated ring containing 3 to 7 carbon atoms. Examples of said cycloalkyl include cyclopropyl, cyclohexyl and cycloheptyl.

Unless otherwise stated or indicated, the term halogen shall mean fluorine, chlorine, or bromine or iodine.

Unless otherwise stated or indicated, the term aryl shall mean an unsubstituted phenyl group, a phenyl group substituted with 1, 2 or 3 substituents each of which being independently loweralkyl, loweralkoxy, halogen, hydroxy, trifluromethyl, phenoxy or benzyloxy.

The phase "each $R_2$" appearing in the definition of group $R_2$ refers to the fact that when n is greater than 1, there are more than one $R_2$ group in the molecule, in which case they may or may not be the same. Similarly, the phase "each $R_3$" appearing in the definition of group $R_3$ refers to the fact that when m is greater than 1, there are more than one $R_3$ group in the molecule in which case they may or may not be the same.

The compounds of this invention are prepared by utilizing the synthetic scheme described below.

In order to simplify the description of the synthetic scheme, the description will be presented with specific reference to the situation where A is $CHR_3$, Y is $CH_2$, $(CHR_2)_n$ is $CHR_2CH_2$ an $R_2$ and $R_3$ together form a methylene bridge, but it will readily be understood that the synthetic scheme can also be applied to the other situations by making obvious modifications where necessary.

Synthetic Scheme

Compounds of formula Ia can be prepared by reacting a 1,4-methano-1,2,3,4-tetrahydro-9-acridinamines of formula II with an aldehyde of formula III where the definitions of X and $R_1$ are as given earlier. Typically, said reaction is conducted in a suitable solvent such as benzene, toluene or xylene at a temperature of about 80-150° C in the presence of a base such as piperidine, morpholine, diethylamine or diisopropylamine.

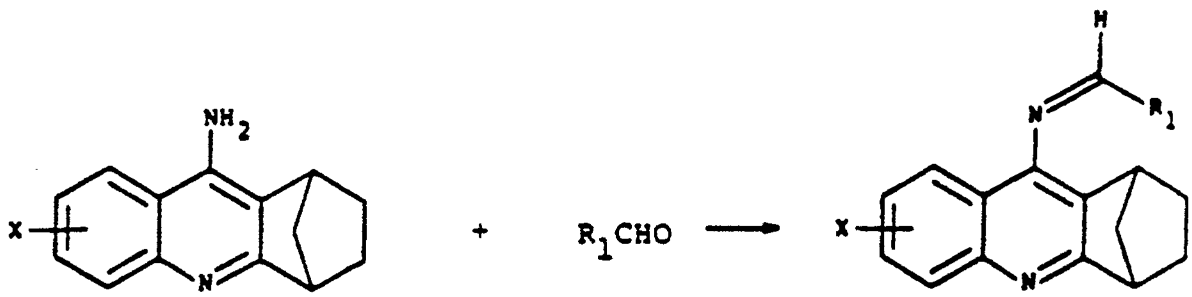

(II) (III) (Ia)

The compounds of Formula I of the present invention are useful in the treatment of various memory dysfunctions characterized by decreased cholinergic function, such as Alzheimer's disease.

This utility is manifested by the ability of these compounds to inhibit the enzyme cholinesterase and thereby increase acetylcholine levels in the brain. Further, the compounds of this invention are in general less toxic and have a broader therapetuic window than heretofore known compounds such as tacrine and physostigmine, making them more therapeutically acceptable.

The ability to inhibit the acetylcholinesterase was determined by the photometric method of Ellman et al., Biochem. Pharmacol. 7,88 (1961). Results of acetylcholinesterase inhibition for some of the compounds of this invention are presented in Table 1 along with those for reference compounds.

Acetylcholinesterase Inhibition Assay

Table 1

| Compound | Acetylcholinesterase Inhibition $IC_{50}$ (molar) |
|---|---|
| 1,4-Methano-N-(phenylmethylene)-1,2,3,4-tetrahydro-9-acridinamine | $1.2 \times 10^{-5}$ |
| 1,4-Methano-N-(phenylmethylene)-6-trifluoromethyl-1,2,3,4-tetrahydro-9-acridinamine | $2.2 \times 10^{-5}$ |
| (Reference Compounds) | |
| Tacrine (9-amino-1,2,3,4-tetrahydroacridine) | $3.1 \times 10^{-7}$ |
| Physostigmine | $6.0 \times 10^{-9}$ |

This utility is further demonstrated by the ability of these compounds to restore cholinergically deficient memory in the Dark Avoidance Assay, where they are in general active over a broader dose range than heretofore known compounds, a distinct therapeutic advantage. In this assay mice are tested for their ability to remember an unpleasant stimulus for a period of 24 hours. A mouse is placed in a chamber that contains a dark compartment; a strong incandescent light drives it to the dark compartment, where an electric shock is administered through metal plates on the floor. The animal is removed from the testing apparatus and tested again, 24 hours later, for the ability to remember the electric shock.

If scopolamine, an anticholinergic that is known to cause memory impairment, is administered before an animal's initial exposure to the test chamber, the animal re-enters the dark compartment shortly after being placed in the test chamber 24 hours later. This effect of scopolamine is blocked by an active test compound, resulting in a greater interval before re-entry into the dark compartment.

The test results are expressed as the percent of a group of animals in which the effect of scopolamine is blocked, as manifested by an increased interval between being placed in the test chamber and re-entering the dark compartment. Results of Dark Avoidance Assay for a representative compound of this invention and physostigmine (reference compound) are presented in Table 2.

Dark Avoidance Assay

Table 2

| Compound | Dose (mg/kg of body weight, s.c) | % of Animals with Scopolamine Induced Memory Deficit Reversal |
|---|---|---|
| 1,4-Methano-N-(phenylmethylene)-1,2,3,4-tetrahydro-9-acridinamine | 0.31 | 27% |
| (Reference Compound) | | |
| Physostigmine | 0.31 | 20% |

Effective quantities of the compounds of the invention may be administered to a patient by any of the various methods, for example, orally as in capsules or tablets, parenterally in the form of sterile solutions or suspensions, and in some cases intravenously in the form of sterile solutions. The free base final products, while effective themselves, may be formulated and administered in the form of their pharmaceutically acceptable acid addition salts for purposes of stability, convenience of crystallization, increased solubility and the like.

Acids useful for preparing the pharmaceutically acceptable acid addition salts of the invention include inorganic acids such as hydrochloric, hydrobromic, sulfuric, nitric, phosphoric and perchloric acids, as well as organic acids such as tartaric, citric, acetic, succinic, maleic, fumaric and oxalic acids.

The active compounds of the present invention may be orally administered, for example, with an inert diluent or with an edible carrier, or they may be enclosed in gelatin capsules, or they may be compressed into tablets. For the purpose of oral therapeutic administration, the active compounds of the invention may be incorporated with excipients and used in the form of tablets, troches, capsules, elixirs, suspensions, syrups, wafers, chewing gum and the like. These preparations should contain at least 0.5% of active compounds, but may be varied depending upon the particular form and may conveniently be between 4% to about 70% of the weight of the unit. The amount of active compound in such compositions is such that a suitable dosage will be obtained. Preferred compositions and preparations according to the present invention are prepared so that an oral dosage unit form contains between 1.0-300 milligrams of active compound.

The tablets, pills, capsules, troches and the like may also contain the following ingredients: a binder such as micro-crystalline cellulose, gum tragacanth or gelatin; an excipient such as starch or lactose, a disintegrating agent such as alginic acid, Primogel, cornstarch and the like; a lubricant such as magnesium stearate or Sterotex; a glidant such as colloidal silicon dioxide; and a sweetening agent such as sucrose or saccharin may be added or a flavoring agent such as peppermint, methyl salicylate, or orange flavoring. When the dosage unit form is a capsule, it may contain, in addition to materials of the above type, a liquid carrier such as a fatty oil. Other dosage unit forms may contain other various materials which modify the physical form of the dosage unit, for example, as coatings. Thus tablets or pills may be coated with sugar, shellac, or other enteric coating agents. A syrup may contain, in addition to the active compounds, sucrose as a sweetening agent and certain preservatives, dyes, coloring and flavors. Materials used in preparing these various compositions should be pharmaceutically pure and non-toxic in the amounts used.

For the purpose of parenteral therapeutic administratin, the active compounds of the invention may be incorporated into a solution or suspension. These preparations should contain at least 0.1% of active compound, but may be varied between 0.5 and about 30% of the weight thereof. The amount of active compound in such compositions is such that a suitable dosage wil be obtained. Preferred compositions and preparations according to the present inventions are prepared so that a parenteral dosage unit contains between 0.5 to 100 milligrams of active compound.

The solutions or suspensions may also include the following components: a sterile diluent such as water for injection, saline solution, fixed oils, polyethylene glycols, glycerine, propylene glycol or other synthetic solvents; antibacterial agents such as benzyl alcohol or methyl parabens; antioxidants such as ascorbic acid or sodium bisulfite; chelating agents such as ethylenediaminetetraacetic acid; buffers such as acetates, citrates or phosphates and agents for the adjustment of tonicity such as sodium chloride or dextrose. The parenteral preparations can be enclosed in disposable syringes or multiple dose vials made of glass or plastic.

Examples of the compounds of this invention include:
N-(phenylmethylene)-3,4-dihydro-1H-thiopyrano[4,3-b]quinolin-10-amine;
N-ethylidene-3,4-dihydro-1H-thiopyrano[4,3-b]quinolin-10-amine;
N-decylidene-3,4-dihydro-1H-thiopyrano[4,3-b]quinolin-10-amine;
N-(phenylmethylene)-3,4-dihydro-1H-pyrano[4,3-b]quinolin-10-amine;
N-[(4-fluorophenyl)methylene-3,4-dihydro-1H-pyrano[4,3-b]quinolin-10-amine;
N-hexylidene-3,4-dihdyro-1H-pyrano[4,3-b]quinolin-10-amine;
N-(phenylmethylene)-2,3-dihydrothieno[3,2-b]quinolin-9-amine;
N-[(2-thienyl)methyene]-2,3-dihydrothieno[3,2-b]quinolin-9-amine;
N-dodecylidene-2,3-dihydrothieno[3,2-b]quinolin-9-amine;
N-(phenylmethylene)-1,3-dihydrothieno[3,4-b]quinolin-9-amine;
1,4-methano-6-methyl-N-(phenylmethylene)-1,2,3,4-tetrahydro-9-acridinamine;
1,4-methano-N-(phenylmethylene)-6-trifluoromethyl-1,2,3,4-tetrahydro-9-acridinamine;
N-[(4-pyridinyl)methylene]-1,3-dihydrothieno[3,4-b]quinolin-9-amine;
N-tetradecylidene-1,3-dihydrothieno[3,4-b]quinolin-9-amine;

1-phenylmethyl-N-(phenylmethylene)-2,3-dihydro-2H-pyrrolo[3,2-b]-quinolin-9-amine;
N-(phenylmethylene)-2,3-dihydro-1H-pyrrolo[3,2-b]quinolin-9-amine;
2-phenylmethyl-N-(phenylmethylene)-1,3-dihydro-2H-pyrrolo[3,4-b]-quinolin-9-amine;
N-(phenylmethylene)-1,3-dihydro-2H-pyrrolo[3,4-b]quinolin-9-amine;
1,4-methano-N-(phenylmethylene)-1,2,3,4-tetrahydro-9-acridinamine;
N-ethylidene-1,4-methano-1,2,3,4-tetrahydro-9-acridinamine;
N-hexadecylidene-1,4-methano-1,2,3,4-tetrahydro-9-acridinamine;
1,4-ethano-N-(phenylmethylene-1,2,3,4-tetrahydro-9-acridinamine;
1,4-ethano-N-[(4-fluorophenyl)methylene]-1,2,3,4-tetrahydro-9-acridinamine; and
N-decylidene-1,4-ethano-1,2,3,4-tetrahydro-9-acridinamine.

The following examples are presented in order to ilustrate this invention.

## EXAMPLE 1

1,4-Methano-N-(phenylmethylene)-1,2,3,4-tetrahydro-9-acridinamine

1,4-Methano-1,2,3,4-tetrahydro-9-acridinamine (8.40 g) was refluxed in 300 mL of toluene which contained 7.0 g of morpholine and 6.4 g of benzaldehyde which had been freshly washed with aqueous $K_2CO_3$. The reaction mixture was refluxed overnight and then concentrated and purified by flash chromatography (20% ethyl acetate/$CH_2Cl_2$) to give 9.51 g of chromatographically pure product. Analytically pure material was obtained by recrystallization from benzene-pentane, mp 128-130 °C.

ANALYSIS:

Calculated for $C_{21}H_{18}N_2$:
84.53%C 6.08%H 9.39%N
Found:
84.49%C 6.02%H 9.31%N

## EXAMPLE 2

N-(Phenylmethylene)-3,4-dihydrothiopyrano-1H-[4,3-b]quinolin-10-amine

3,4-Dihydrothiopyrano-1H-[4,3-b]quinolin-10-amine (8.64 g) was suspended in 300 mL of toluene to which morpholine (7.0 g) and benzaldehyde (The benzaldehyde was freshly washed with aqueous $K_2CO_3$ solution.) were then added. This reaction mixture was then refluxed overnight with the separation of $H_2O$ (Dean-Stark trap) and then concentrated and purified by flash chromatography (20% ethyl acetate/$CH_2Cl_2$). The product-containing fractions were concentrated to give 7.30 g of chromatographically pure product, mp 171-173 °c. Analytically pure material was obtained by recrystallization from $CH_2Cl_2$/pentane, mp 175-176 °C.

ANALYSIS:

Calculated for $C_{19}H_{16}N_2S$:
74.96%C 5.30%H 9.20%N
Found:
74.97%C 5.25%H 9.18%N

## Example 3

### 1,4-Methano-6-methyl-1,2,3,4-tetrahydro-9-acridinamine

A solution of 2-amino-4-methylbenzonitrile (16.0 g) and zinc chloride (24.7 g) in 70 ml of nitrobenzene was heated at 50°C for 1 hour. To this was added norcamphor (20.0 g) and the mixture was stirred at 130°C for 3 hours. The reaction mixture was cooled and diluted with ether and the zinc complex was filtered. This complex was partitioned between aqueous $NH_4OH$ and 2-butanone (MEK) and the aqueous phase was extracted with MEK. The organics were washed with water, dried (saturated NaCl, $MgSO_4$) and concentrated to an oil which was triturated with ether to give 12.8 g of white powder, m.p. 159-162°C. A 4.0 g portion was recrystallized from isopropyl ether to give 2.5 g of analytically pure white solid, m.p. 162-164°C.

ANALYSIS:

Calculated for $C_{15}N_{16}N_2$:
80.32%C 7.19%H 12.49%N
Found:
80.29%C 7.05%H 12.52%N

## Example 4

### 1,4-Methano-6-methyl-N-(phenylmethylene)-1,2,3,4-tetrahydro-9-acridinamine

A solution prepared from 1,4-methano-6-methyl-1,2,3,4-tetrahydro-9-acridinamine (8.6 g), benzaldehyde (6.3 g, freshly washed with $K_2CO_3$ solution), morpholine (6.9 ml) and toluene (300 ml) was refluxed, with removal of water, for eighteen (18) hours. At this time, 6.3 g of benzaldehyde was added and reflux was continued for twenty-four (24) hours. The solvent was then removed in vacuo and the imine was purified via flash chromatography (DCM) to give 7.3 g of an organish solid, m.p. 144-147°C. A 3.5 g portion was recrystallized from isopropyl ether to give 2.54 g of light yellow crystals, m.p. 149-152°C.

ANALYSIS:

Calculated for $C_{22}H_{20}N_2$:
84.58%C 6.45%H 8.97%N
Found:
84.46%C 6.42%H 9.00%N

## Example 5

### 1,4-Methano-1,2,3,4-tetrahydro-6-trifluoromethyl-9-acridinamine

To a solution of 2-amino-4-trifluoromethylbenzonitrile (12.8 g) in 50 ml of nitrobenzene was added freshly fused and pulverized $ZnCl_2$ (14.1 g). This was heated at 50°C for 1 hour and to this mixture was added norcamphor (11.4 g). The reaction mixture was heated at 130°C for 3 hours, after which it was

cooled, diluted with ethyl ether and filtered. The resulting solid was partitioned between 2-butanone (MEK) and aqueous $NH_4OH$ and the aqueous portion was extracted with MEK. The combined organics were washed with water, dried (saturated NaCl, $MgSO_4$) and concentrated to a solid which was triturated with ether/hexane to give 10.3 g of a white powder, m.p. 174-179° C. A 4.0 g portion was recrystallized from methanol/water to give 3.5 g of an analytically pure white powder, m.p. 175-178° C.

ANALYSIS:

Calculated for $C_{15}H_{13}F_3N_2$:
64.74%C 4.71%H 10.07%N
Found:
64.70% 4.88%H 10.09%N

## Example 6

### 1,4-Methano-N-(phenylmethylene)-6-trifluoromethyl-1,2,3,4-tetrahydro-9-acridinamine

A mixture prepared from 1,4-methano-1,2,3,4-tetrahydro-6-trifluoromethyl-9-acridinamine (7.65 g), benzaldehyde (4.4 g, freshly washed with $K_2CO_3$), morpholine (4.8 ml) and toluene (300 ml) was refluxed with removal of water for eighteen (18) hours. At this point, an additional 4.4 g of benzaldehyde was added and reflux was continued for twenty-four (24) hours.

The reaction was then concentrated to a solid and the residue was chromatographed (DCM) to give 8.3 g of an orangish solid, m.p. 120-126° C. A 4.07 g portion was recrystallized from cyclohexane to give 2.56 g of an off-white solid, m.p. 127-130° C.

ANALYSIS:

Calculated for $C_{22}H_{17}F_3N_2$:
72.12%C 4.68%H 7.65%N
Found:
72.15%C 4.83%H 7.61%N

## Example 7

### 3,4-Dihydro-1H-thiopyrano[4,3]quinolin-10-amine

Tetrahydrothiopyran-4-one (10.0 g) was mixed with anthranilonitrile (5.08 g) and the mixture warmed at 60° C until a homogeneous solution was obtained. Freshly fused $ZnCl_2$ (8.2 g) was then added portionwise and the temperature of the reaction mixture raised to 120° C. After two (2) hours it was cooled and distributed between 10% NaOH and 2-butanone. The organic phase was separated, dried and concentrated, and the crude product triturated with $Et_2O$ and then passed over a silica gel column (5% $Et_3N$/ethyl acetate). The product-containing fractions were concentrated and the product recrystallized from toluene to give 3.66 g, m.p. 214-216° C.

ANALYSIS:

Calculated for $C_{12}H_{12}N_2S$:
66.63%C 5.59%H 12.95%N
Found:
66.74% 5.71%H 12.79%N

Example 8

N-(Phenylmethylene)-3,4-dihydrothiopyrano-1H-[4,3-b]quinolin-10-amine

3,4-Dihydrothiopyrano-1H-[4,3-b]quinolin-10-amine (8.64 g) was suspended in 300 ml of toluene to which morpholine (7.0 g) and benzaldehyde (freshly washed with aqueous $K_2CO_3$ solution, 8.50 g) were then added. The reaction mixture was refluxed overnight with removal of water (Dean-Stark trap) and then concentrated and purified by flash chromatography (20% $EtOAc/CH_2Cl_2$). The product-containing fractions were concentrated to give 7.30 g of chromatographically pure product, m.p. 171-173° C. Analytically pure material was obtained by recrystallization from $CH_2Cl_2$/pentane, m.p. 175-176° C.

ANALYSIS:

Calculated for $C_{19}H_{16}N_2S$:
74.96%C 5.30%H 9.20%N
Found:
74.97%C 5.25%H 9.18%N

Example 9

1,4-Dihydro-1,4-ethano-9-acridinamine

A mixture prepared from anthranilonitrile (4.18 g), freshly fused zinc chloride (7.2 g) and 15 ml of nitrobenzene was heated at 50° C for 45 minutes. To the resulting suspension was added bicyclo[2.2.2]oct-2-en-5-one (6.5 g) and this was heated at 130° C for 1.5 hours.

The reaction mixture was cooled and treated with ethyl ether, and the precipitate was filtered, rinsed with ether and then partitioned between 2-butanone (MEK) and aqueous $NH_4OH$ solution. The aqueous phase was extracted with MEK and the combined organics were washed with water and dried (saturated NaCl, $MgSO_4$). Removal of the solvents gave 5.7 g of an off-white powder which was recrystallized from methanol/water to give 4.76 of an off-white solid, m.p. 218-220° C d.

ANALYSIS:

Calculated for $C_{15}H_{14}N_2$:
81.05%C 6.35%H 12.60%N
Found:
80.96%C 6.34%H 12.65%N

Example 10

1,4-Dihydro-1,4-ethano-N-(phenylmethylene)-9-acridinamine

A mixture of 1,4-dihydro-1,4-ethano-9-acridinamine (11.4 g), benzaldehyde (8.2 g, freshly washed with $K_2CO_3$ solution) and morpholine (9.0 ml) in 350 ml toluene was refluxed with removal of water for eighteen (18) hours. An additional 6 g of benzaldehyde was added and reflux was continued for twelve (12) hours with removal of water.

The reaction mixture was concentrated, passed through a column of florisil (DCM) and the imine was purified via flash chromatography (DCM then 5% EtOAc/DCM) to give 13.4 g of an orangish solid. A 3.0 g portion was recrystallized from methanol/water to give 2.54 g of light yellow crystals, m.p. 178-180° C

ANALYSIS:

Calculated for $C_{22}H_{18}N_2$:
85.13%C 5.85%H 9.02%N
Found:
85.03% 6.05%H 8.94%N

**Claims**

1. A compound of the formula I

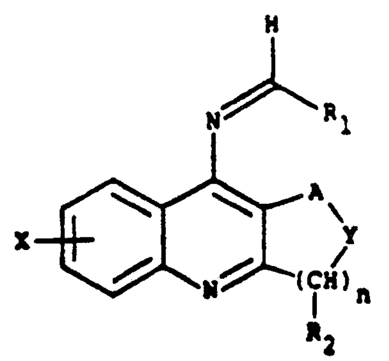

I

wherein n is 1-4; $R_1$ is hydrogen, alkyl, aryl, arylloweralkyl, naphthyl, furyl, thienyl, pyridinyl or pyrrolyl; A is a direct bond or $(CHR_3)_m$, m being 1-3; X is hydrogen, loweralkyl, cycloalkyl, loweralkoxy, halogen, hydroxy, nitro, trifluoromethyl, formyl, loweralkylcarbonyl, arylcarbonyl, -SH, loweralkylthio, $-NHCOR_4$ or $-NR_5R_6$, $R_4$ being hydrogen or loweralkyl, and $R_5$ and $R_6$ being independently hydrogen, loweralkyl or cycloalkyl; Y is $CH_2$, O, S or $NR_7$; and each $R_2$, each $R_3$ and $R_7$ are independently hydrogen, loweralkyl or arylloweralkyl or taken two at a time form a methylene or ethylene bridge constituting a part of a five-membered or larger ring, with the proviso that when Y is $CH_2$, $R_2$ and $R_3$ must together constitute a methylene or ethylene bridge; a stereo, optical or geometrical isomer thereof, or a pharmaceutically acceptable acid addition salt thereof.

2. A compound as defined in claim 1, wherein A is $-CHR_3$, Y is $-CH_2$,

$$-\underset{R_2}{(CH)}_n \text{ is } -\underset{R_2}{CH}-CH_2-$$

and $R_2$ and $R_3$ together form a methylene bridge.

3. A compound as defined in Claim 1, where A is $CH_2$, Y is S, $R_2$ is hydrogen and n is 2.

4. A compound as defined in Claim 2 or 3, wherein $R_1$ is aryl.

5. A compound as defined in claims 1 - 4, where X is hydrogen, loweralkyl or trifluoromethyl.

6. The compound as defined in claim 1 which is 1,4-methano-N-(phenylmethylene)-1,2,3,4-tetrahydro-9-acridinamine.

7. The compound as defined in claim 1 which is 1,4-methano-N-(phenylmethylene)-6-trifluoromethyl-1,2,3,4-tetrahydro-9-acridin-amine.

8. A pharmaceutical composition which comprises as the active ingredient a compound as defined in claim 1 and a suitable carrier therefor.

9. Use of a compound for the preparation of a medicament being effective in the treatment of memory dysfunctions characterized by decreased cholinergic function.

10. A process for the preparation of a compound as defined in claim 1, which comprises reacting a compound of the formula II

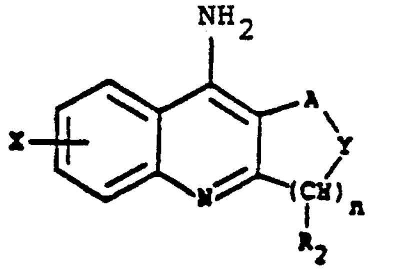

II

where A, Y, X, $R_2$ and n are as defined in claim 1, with an aldehyde of the formula $R_1CHO$, where $R_1$ is as defined in claim 1.

11. A process as defined in claim 9 where A is $CHR_3$, Y is $CH_2$, the radical

$$-\underset{R_2}{(CH)_n}- \text{ is } -\underset{R_2}{CH}-CH_2-,$$

and $R_2$ and $R_3$ together form a methylene bridge.

Claims for the following contracting states: ES, GR

1. A process for the preparation of a compound of the formula I

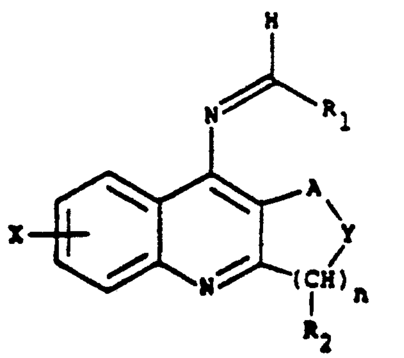

I

wherein n is 1-4; $R_1$ is hydrogen, alkyl, aryl, arylloweralkyl, naphthyl, furyl, thienyl, pyridinyl or pyrrolyl; A is a direct bond or $(CHR_3)_m$, m being 1-3; X is hydrogen, loweralkyl, cycloalkyl, loweralkoxy, halogen, hydroxy, nitro, trifluoromethyl, formyl, loweralkylcarbonyl, arylcarbonyl, -SH, loweralkylthio, $-NHCOR_4$ or $-NR_5R_6$, $R_4$ being hydrogen or loweralkyl, and $R_5$ and $R_6$ being independently hydrogen, loweralkyl or cycloalkyl; Y is $CH_2$, O, S or $NR_7$; and each $R_2$, each $R_3$ and $R_7$ are independently hydrogen, loweralkyl or arylloweralkyl or taken two at a time form a methylene or ethylene bridge constituting a part of a five-membered or larger ring, with the proviso that when Y is $CH_2$, $R_2$ and $R_3$ must together constitute a methylene or ethylene bridge; a stereo, optical or geometrical isomer thereof, or a pharmaceutically acceptable acid addition salt thereof,
which comprises reacting a compound of the formula II

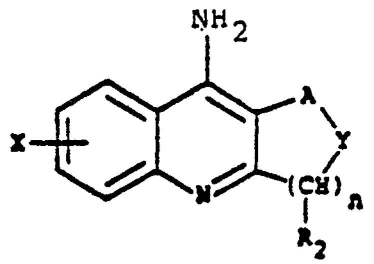

II

where A, Y, X, $R_2$ and n are as defined above, with an aldehyde of the formula $R_1CHO$, where $R_1$ is as defined above.

2. A process as defined in claim 1 where A is $-CHR_3$, Y is $-CH_2$, the radical

$$-\underset{R_2}{(CH)_n}- \text{ is } -\underset{R_2}{CH}-CH_2-,$$

and $R_2$ and $R_3$ together form a methylene bridge.

3. A process as defined in claim 1 where A is $-CH_2$, Y is S, $R_2$ is hydrogen and n is 2.

4. A process as defined in claims 1 to 3, where $R_1$ is aryl.

5. A process as defined in claims 1 to 4, where X is hydrogen, loweralkyl or trifluoromethyl.

6. The process as defined in claim 2 wherein 1,4-methano-N-(phenylmethylene)-1,2,3,4-tetrahydro-9-acridinamine is prepared.

7. The process as defined in claim 2 wherein 1,4-methano-N-(phenylmethylene)-6-trifluoromethyl-1,2,3,4-tetrahydro-9-acridinamine is prepared.

8. Use of a compound as defined in claim 1 for the preparation of a medicament being effective in the treatment of memory dysfunctions characterized by decreased cholinergic function.

European Patent Office

# EUROPEAN SEARCH REPORT

Application number: EP 88114249.1

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | EP - A2 - 0 179 383 (HOECHST-ROUSSEL PHARMACEUTICAL INC.)<br>* Claims 1,10,11,13; page 14, lines 6-19 *<br>-- | 1,8-10 | C 07 D 219/10<br>C 07 D 221/16<br>C 07 D 221/22<br>C 07 D 495/04<br>A 61 K 31/47<br>//(C 07 D 495/04<br>C 07 D 219:00<br>C 07 D 333:00)<br>(C 07 D 495/04<br>C 07 D 219:00<br>C 07 D 335:00) |
| A | CHEMICAL ABSTRACTS, vol. 103, no. 7, August 15, 1985, Columbus, Ohio, USA<br>FLOOD, J.F.; SMITH, G.E.; CHERKIN, A. "Memory enhancement: supra-additive effect of subcutaneous cholinergic drug combinations in mice."<br>page 45, column 1, abstract-no. 48 119t<br>& Psychopharmacology (Berlin) 1985, 86 (1-2), 61-7<br>-- | 1,9 | |
| | | | **TECHNICAL FIELDS SEARCHED (Int. Cl.4)** |
| A | CHEMICAL ABSTRACTS, vol. 100, no. 15, April 09, 1984, Columbus, Ohio, USA<br>CHERKIN, A.; FLOOD, J.F. "Remarkable potentiation among memory-enhancing cholinergic drugs in mice"<br>page 50, column 1, abstract-no. 114 871y<br>& Mod. Aging Res. 1983, 3A (Intervent. Aging Process, PT.A), 225-45<br>---- | 1,9 | C 07 D 219/00<br>C 07 D 221/00<br>C 07 D 495/00 |

The present search report has been drawn up for all claims

| Place of search | Date of completion of the search | Examiner |
|---|---|---|
| VIENNA | 13-12-1988 | BRUS |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO Form 1503. 03.82